# EUROPÄISCHE PATENTANMELDUNG

(11) **EP 2 357 461 A1**
(43) Veröffentlichungstag der Anmeldung: **17.08.2011**
(21) Anmeldenummer: 11151506.0
(22) Anmeldetag: 20.01.2011
(51) Int. Cl.: G01N 1/22

(54) **Behälter mit einem Trägermaterial-Element, das dazu ausgelegt ist, einen Inhaltsstoff einer Probe aufzunehmen**

(30) Priorität: 17.02.2010 DE 202010002462 U
(71) Anmelder: MWT Mikrowellen Labor Technik AG, 9435 Heerbrugg (CH)
(72) Erfinder: Lautenschläger, jens, CH-9435 Heerbrugg / SG (CH); Lautenschläger, Werner, CH-9435 Heerbrugg / SG (CH)
(74) Vertreter: Rupp, Christian

(57) **Zusammenfassung**

Die Erfindung betrifft einen Behälter mit einem Trägermaterial Element (8), das dazu ausgelegt ist, einen Inhaltsstoff einer gasförmigen oder flüssigen Probe aufzunehmen, aufweisend ein Gehäuse (2) mit einer ersten Öffnung (4), die dafür vorgesehen ist, die Probe in das Gehäuse (2) einzuführen und mit einer zweiten Öffnung (6), die dafür vorgesehen ist, die Probe zumindest teilweise in rückhaltbarer Konzentration wieder aus dem Gehäuse (2) auszuführen,wobei das Trägermaterial-Element (8) in dem Gehäuse (2) angeordnet ist,gekennzeichnet durch ein erstes Filterelement (10) und ein zweites Filterelement (12), wobei das erste Filterelement (10), das zweite Filterelement (12) und das Trägermaterial-Element (8) derart in dem Gehäuse (2) angeordnet sind, dass die durch die erste Öffnung (4) eingeführte Probe ausschließlich nach einem Durchströmen des ersten Filterelements (10), des Trägermaterial-Elements (8) und des zweiten Filterelements (12) durch die zweite Öffnung (6) das Gehäuse (2) verlassen kann.

## Beschreibung

Die Erfindung betrifft einen Behälter mit einem Trägermaterial-Element, das dazu ausgelegt ist, einen Inhaltsstoff einer gasförmigen oder flüssigen Probe aufzunehmen. Außerdem betrifft die Erfindung eine Vorrichtung zur Bestimmung des Inhaltsstoffes der Probe mithilfe eines solchen Behälters.

Quecksilber ist unter den Metallen eines der mobilsten Elemente und verteilt sich somit zunehmend auf der Erdoberfläche und in der Atmosphäre. Die größten Mengen an Quecksilber werden neben vulkanischen Aktivitäten durch den Menschen freigesetzt. Durch die Verbrennung fossiler Brennstoffe und durch thermische Verfahren wie Erzaufbereitung für die Metall- und Stahlproduktion, sowie Verarbeitung von keramischen Rohstoffen usw. wird Quecksilber in Umlauf gebracht. Die gebildeten Quecksilber-Verbindungen werden durch Bioakkumulation in Lebewesen angereichert und gelangen so in Form von hochtoxischem Methylquecksilber, vor allem durch den Verzehr von Fischen und Meeresprodukten, in den Nahrungskreislauf. Somit sind Quecksilberkontrollen in gasförmigen und flüssigen Proben von zunehmender Bedeutung.

Aus der Literatur ("Dem Quecksilber auf der Spur", Lautenschläger, W.; McKinney, C. , Laborpraxis-Würzburg, 2004, 28, 6, Seiten 30 bis 32) ist ein System mit der Bezeichnung "DMA-80" bekannt, mit dem der Quecksilbergehalt in flüssigen und festen Proben ohne vorherige chemische Aufbereitung in kurzer Zeit bestimmt werden kann. Hierzu werden Proben bei hoher Temperatur in einem Verbrennungsraum katalytisch im Sauerstoffstrom verbrannt und das Quecksilber als Goldamalgam angereichert. Anschließend wird das gesammelte Quecksilber durch spontanes Atomisieren spektroskopisch bestimmt.

Bei dem System "DMA-80" wird zunächst ein "Proben-Schiffchen", im Folgenden auch kurz "Schiffchen" genannt, mit der festen oder flüssigen Probe beladen bzw. befüllt und anschließend wird das so beladene Schiffchen in den Verbrennungsraum eingeführt. Eine gasförmige Probe lässt sich auf diese Weise nicht untersuchen.

Der Erfindung liegt die Aufgabe zugrunde, ein Hilfsmittel bzw. eine Vorrichtung anzugeben, mit dem bzw. mit der ein Inhaltsstoff einer gasförmigen Probe vergleichsweise einfach ermittelt werden kann.

Diese Aufgabe wird gemäß der Erfindung mit den in den unabhängigen Ansprüchen genannten Gegenständen gelöst. Besondere Ausführungsarten der Erfindung sind in den abhängigen Ansprüchen angegeben.

Gemäß der Erfindung ist ein Behälter mit einem Trägermaterial-Element vorgesehen, das dazu ausgelegt ist, einen Inhaltsstoff einer gasförmigen oder flüssigen Probe aufzunehmen; der Behälter weist ein Gehäuse mit einer ersten Öffnung auf, die dafür vorgesehen ist, die Probe in das Gehäuse einzuführen und mit einer zweiten Öffnung, die dafür vorgesehen ist, die Probe zumindest teilweise in rückhaltbarer Konzentration wieder aus dem Gehäuse auszuführen; das Trägermaterial-Element ist dabei in dem Gehäuse angeordnet. Weiterhin weist der Behälter ein erstes Filterelement und ein zweites Filterelement auf, wobei das erste Filterelement, das zweite Filterelement und das Trägermaterial-Element derart in dem Gehäuse angeordnet sind, dass die durch die erste Öffnung eingeführte Probe ausschließlich nach einem Durchströmen des ersten Filterelements, des Trägermaterial-Elements und des zweiten Filterelements durch die zweite Öffnung das Gehäuse verlassen kann.

Mit dem in dem Behälter befindlichen Trägermaterial-Element lässt sich der Inhaltsstoff der Probe auffangen, indem die Probe durch die erste Öffnung in den Behälter eingeführt bzw. eingeleitet wird. Anschließend lässt sich der so aufgefangene Inhaltsstoff analysieren, zum Beispiel mittels Verbrennen unter Sauerstoff in einem Verbrennungsraum.

Vorzugsweise handelt es sich bei dem Inhaltsstoff um Quecksilber, so dass das Trägermaterial-Element dazu ausgelegt ist, Quecksilber aus der Probe aufzunehmen. Hierdurch wird ermöglicht, den Quecksilbergehalt einer gasförmigen oder flüssigen Probe zu bestimmen.

Vorzugsweise ist das Trägermaterial-Element dazu ausgelegt, aus der Probe Quecksilber in Form von Quecksilberatomen und/oder in Form einer Quecksilberverbindung aufzunehmen.

Vorzugsweise weist das Trägermaterial-Element ein Trägermaterial in Form von Graphit und/oder einen Amalgam-Bildner auf. Dieses Material eignet sich besonders gut dazu, Quecksilber zu adsorbieren bzw. anzureichern.

Vorzugsweise besteht das Gehäuse aus einem sauerstoffresistentem Material, insbesondere aus Quarz, Keramik oder Metall. Auf diese Weise eignet sich der Behälter für eine Halterung des Trägermaterial-Elements während einer Verbrennung unter Sauerstoff zur Analyse des Inhaltsstoffs.

Vorzugsweise besteht das Gehäuse aus einem Material, das bis 900°C, vorzugsweise bis 1000°C temperaturresistent ist. Auf diese Weise eignet sich der Behälter für eine Halterung des Trägermaterial-Elements während einer Verbrennung bei einer entsprechend hohen Temperatur zur Analyse des Inhaltsstoffs.

Vorzugsweise weisen das erste Filterelement und/oder das zweite Filterelement Quarzfasern und/oder Keramikfasern auf; das erste Filterelement und/oder das zweite Filterelement können auch vorteilhaft aus Quarzfaserwolle und/oder Keramikfaserwolle bestehen. Hierdurch wird eine besonders kontrollierte, verzögerte Verbrennung des auf dem Trägermaterial angereicherten bzw. adsorbierten Inhaltsstoffes ermöglicht, so dass sich hierbei insbesondere eine unerwünschte, explosionsartige Reaktion vermeiden lässt, die mit der Gefahr eines unerwünschten Materialverlustes verbunden wäre. Außerdem lässt sich so eine vergleichsweise große Menge des Inhaltstoffes für eine kontrollierte, verzögerte Verbrennung in dem Behälter aufnehmen. Das ist von Vorteil mit Bezug auf die Nachweisgrenze bei der Analyse; die erzielbare Empfindlichkeit lässt sich hierdurch erhöhen. Insbesondere lässt sich hierdurch auch eine extrem geringe, störungsfreie Nachweisgrenze ermöglichen.

Vorzugsweise bestehen das erste Filterelement und/oder das zweite Filterelement aus einem Material, das bis 900°C, vorzugsweise bis 1000°C temperaturresistent ist. Hierdurch eignet sich der Behälter besonders gut für eine Halterung des Trägermaterial-Elements während einer Verbrennung bei einer entsprechend hohen Temperatur zur Analyse des Inhaltsstoffs.

Vorzugsweise ist das Trägermaterial-Element zwischen dem ersten Filterelement und dem zweiten Filterelement angeordnet.

Vorzugsweise weist der Behälter außerdem wenigstens ein weiteres Filterelement auf.

Gemäß einem weiteren Aspekt der Erfindung ist eine Vorrichtung zur Bestimmung eines Inhaltsstoffes einer Probe vorgesehen, die einen beheizbaren Verbrennungsraum und einen erfindungsgemäßen Behälter aufweist, wobei der Behälter und der Verbrennungsraum derart dimensioniert sind, dass sich der Behälter vollständig in den Verbrennungsraum einführen lässt. Vorteilhaft ist die Vorrichtung dabei dazu ausgebildet, das in den Verbrennungsraum eingebrachte Trägermaterial-Element unter Sauerstoffzufuhr zu verbrennen und ein dabei entstehendes Pyrolysegas zu analysieren.

Die Erfindung wird im Folgenden anhand eines Ausführungsbeispiels und mit Bezug auf die Zeichnungen näher erläutert. Es zeigen:
- Fig. 1: einen Drehteller eines automatischen Probenwechslers mit einem erfindungsgemäßen Behälter,
- Fig. 2: den Drehteller des automatischen Probenwechslers, angeordnet in einer Vorrichtung zur Bestimmung eines Inhaltsstoffes einer Probe,
- Fig. 3: ein automatisches Zuführen des Behälters von dem Drehteller in einen Verbrennungsraum eines Ofens der Vorrichtung mithilfe einer Gabel eines Transportarms und
- Fig. 4: ein Einschieben des Behälters in den Verbrennungsraum des Ofens für eine temperaturprogrammierbaren Verbrennung.

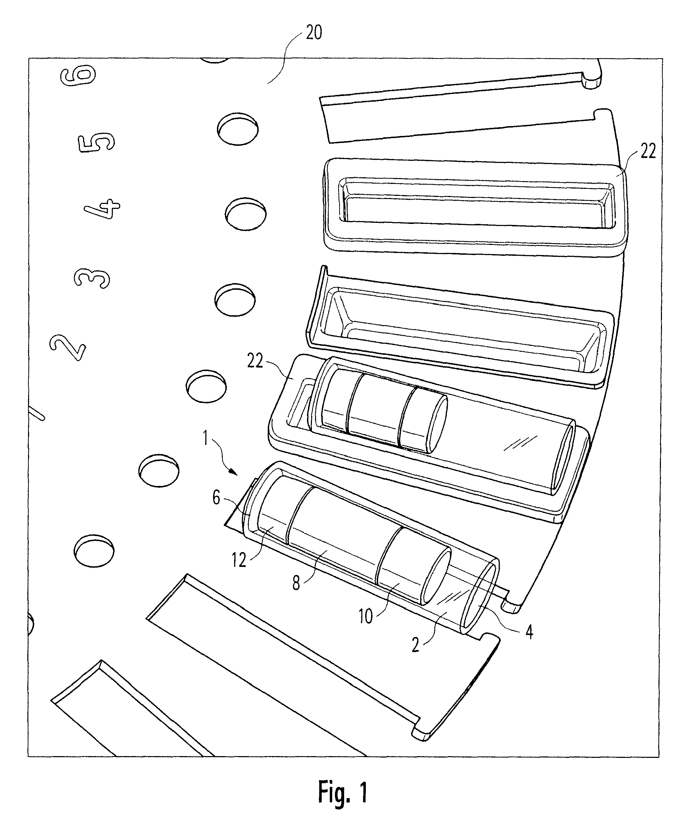

In Fig. 1 ist ein Ausführungsbeispiel eines erfindungsgemäßen Behälters 1 mit einem Trägermaterial-Element 8 gezeigt. Der Behälter 1 eignet sich als Hilfsmittel bei einer Bestimmung eines Inhaltsstoffs einer gasförmigen oder flüssigen Probe bzw. eines Fluids.

Das Trägermaterial-Element 8 ist dazu ausgelegt, den Inhaltsstoff der gasförmigen oder flüssigen Probe aufzunehmen, insbesondere durch Adsorbieren. Bei der Probe kann es sich beispielsweise um ein Pyrolysegas, um ein Abwasser oder um ein Lösungsmittel handeln. Das Pyrolysegas kann aus einer Verbrennung einer festen Probe gebildet sein, so dass mit dem Behälter 1 als Hilfsmittel indirekt auch der Inhaltstoff einer festen Probe bestimmt werden kann.

Bei dem Inhaltsstoff kann es sich um Quecksilber handeln. Insbesondere kann das Trägermaterial-Element 8 dafür ausgebildet sein, Quecksilber aus der Probe in Form von Quecksilberatomen und/oder einer Quecksilberverbindung aufzunehmen.

Der Behälter 1 weist ein Gehäuse 2 auf, in dem das Trägermaterial-Element 8 angeordnet ist. Beispielsweise kann das Gehäuse 2 rohrförmig ausgebildet sein. In diesem Sinne kann der Behälter 1 als "Adsorptionsröhrchen" bezeichnet werden.

Das Gehäuse 2 weist eine erste Öffnung 4 auf, die dafür vorgesehen ist, die Probe in das Gehäuse 2 einzuführen und eine zweite Öffnung 6, die dafür vorgesehen ist, die Probe zumindest teilweise wieder aus dem Gehäuse 2 auszuführen. Beispielsweise kann vorgesehen sein, dass die Probe durch die erste Öffnung 4 in das Gehäuse 2 hinein geleitet und durch die zweite Öffnung 6 aus dem Gehäuse 2 heraus geleitet wird, wobei während des Durchströmens der gasförmigen bzw. flüssigen Probe bzw. des Fluids der Inhaltsstoff von dem Trägermaterial-Element 8, beispielswiese durch Adsorption, aufgenommen wird, so dass er sich dort anreichert.

Das Gehäuse 2 besteht vorzugsweise aus einem sauerstoffresistentem Material, beispielsweise aus Quarz, Keramik oder Metall. Hierdurch lässt sich vorteilhaft nach Anreicherung des Inhaltsstoffs das in dem Behälter 1 befindliche Trägermaterial-Element 8 für eine Analyse in einen Verbrennungsraum unter Sauerstoffzufuhr verbrennen. Dementsprechend weiterhin vorteilhaft besteht das Gehäuse 2 aus einem Material, das bis 900°C, vorzugsweise bis 1000°C temperaturresistent ist.

Weiterhin weist der Behälter 1 ein erstes Filterelement 10 und ein zweites Filterelement 12 auf. Das erste Filterelement 10, das zweite Filterelement 12 und das Trägermaterial-Element 8 sind dabei derart in dem Gehäuse 2 angeordnet, dass die durch die erste Öffnung 4 eingeführte Probe ausschließlich nach einem Durchströmen des ersten Filterelements 10, des Trägermaterial-Elements 8 und des zweiten Filterelements 12 durch die zweite Öffnung 6 das Gehäuse 2 verlassen kann. Vorzugsweise ist das Trägermaterial-Element 8 zwischen dem ersten Filterelement 10 und dem zweiten Filterelement 12 angeordnet. Das Trägermaterial-Element 8 kann also zwischen den beiden Filterelementen 10, 12 eingeschlossen in dem Gehäuse 2 angeordnet sein.

Vorzugsweise bestehen die Filterelemente 10, 12 aus entsprechend temperaturresistentem Material, also insbesondere aus einem Material, das bis 900°C, vorzugsweise bis 1000°C temperaturresistent ist.

Beispielsweise eignet sich hierfür ein keramischer Werkstoff, wie ein Oxid oder Quarz, vorzugsweise mit großer Oberfläche und reduzierter Diffusion.

Durch die beiden Filterelemente 10, 12 lässt sich eine Verbrennung des Inhaltsstoffs in einem Sauerstoffstrom zur Analyse erzielen, die besonders kontrolliert und verzögert abläuft bzw. erfolgt. Insbesondere lässt sich eine explosionsartige Reaktion vermeiden; auch ein schlagartiges Zucken lässt sich hierdurch vermeiden.

Außerdem sind die Filterelements 10, 12 vorteilhaft dazu ausgebildet, Staubpartikel, beispielsweise aus Rauch, abzufangen bzw. zurückzuhalten.

Vorzugsweise weist das Trägermaterial-Element 8 ein Trägermaterial auf, bei dem es sich beispielsweise um Graphit, insbesondere um hochaktiven Graphit oder um Aktivkohle handeln kann. Bei dem Trägermaterial kann es sich auch um einen Amalgam-Bildner handeln, beispielsweise auf Gold- oder Silberbasis. Hierdurch lässt sich mit dem Trägermaterial-Element 8 aus einer gasförmigen oder flüssigen Probe stammendes Quecksilber bzw. eine aus der Probe stammende Quecksilberverbindung besonders effektiv festhalten bzw. anreichern.

Ein Verfahren zur Bestimmung eines Inhaltsstoffs einer Probe mithilfe eines erfindungsgemäßen Behälters kann folgende Schritte aufweisen:
(a) Die Probe wird durch den Behälter 1 hindurch geleitet, so dass sich der Inhaltsstoff der Probe an dem Trägermaterial-Element 8 anreichert.
(b) Der Behälter 1 wird nach Schritt (a) in einen Verbrennungsraum gegeben und dort unter Sauerstoffzufuhr verbrannt, so dass ein Pyrolysegas entsteht.
(c) Nach Schritt (b) wird der Inhaltsstoff durch Analyse des Pyrolysegases bestimmt.

Durch den Behälter 1 lässt sich die Position des Trägermaterials und des Inhaltsstoffs im Verbrennungsraum fixieren.

Nach Schritt (a) und vor Schritt (b) kann vorgesehen sein, dass - wie in Fig. 1 gezeigt - der Behälter 1 auf einen Drehteller 20 eines vorzugsweise automatischen Probenwechslers gelegt wird. Der Behälter 1 kann dabei auf einem Schiffchen 22 aus Quarz abgelegt sein, wie in Fig. 1 unter Positionsnummer "2" auf dem Drehteller 20 exemplarisch gezeigt. Das Schiffchen kann auch aus Metall bestehen, wie in Fig. 1 unter Positionsnummer "3" auf dem Drehteller 20 exemplarisch (ohne Behälter) gezeigt. Alternativ kann vorgesehen sein, dass der Behälter 1 direkt, also ohne Schiffchen, auf den Drehteller 20 gelegt wird, wie unter Positionsnummer "1" gezeigt.

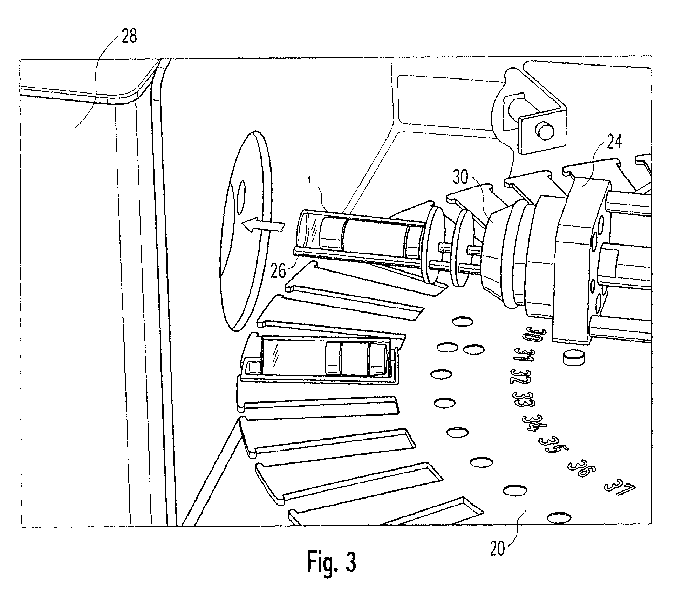

Bei Schritt (b) kann vorgesehen sein, dass der Behälter 1 manuell oder automatisch in den Verbrennungsraum gegeben wird. Beispielswiese kann vorgesehen sein, wie in Fig. 3 angedeutet, dass sich der Behälter 1 von dem Drehteller 20 von unten durch einen (in der Fig. 3 nicht gezeigten) Stößel angehoben wird, worauf eine Gabel 26 eines Transportarms 24 den Behälter 1 übernimmt und dann in den Verbrennungsraum einführt, der in einem Ofen 28 einer entsprechenden Vorrichtung angeordnet ist. Es kann auch vorgesehen sein, dass der Behälter 1 manuell auf die Gabel 26 aufgelegt wird und anschließend mithilfe des Transportarms 24 in den Verbrennungsraum eingebracht wird.

Bei Schritt (b) wird die Verbrennung vorzugsweise mithilfe eines Temperaturprogramms mit zeitdefinierter Zersetzung des Inhaltsstoffs durchgeführt. Die Verbrennung kann beispielswiese bei einer Temperatur zwischen Raumtemperatur und 1000°C vorgesehen sein.

Bei der Verbrennung in dem Verbrennungsraum wird nur Sauerstoff zugeführt, da der zu untersuchende Inhaltsstoff bereits in dem Behälter 1 in den Verbrennungsraum eingebracht worden ist. Bei der Verbrennung wird dieses Material bzw. der Inhaltsstoff dann aufgrund der Filterelemente 10, 12 wie oben bereits dargestellt langsam und kontrolliert zersetzt.

Der Verbrennungsraum weist vorzugsweise einen ersten Heizbereich und einen zweiten Heizbereich auf, wobei die in Schritt (b) erfolgende Verbrennung zunächst in dem ersten Heizbereich unter Sauerstoffzufuhr erfolgt und ein dabei entstandenes Verbrennungsgas anschließend in dem zweiten Heizbereich vollständig zu atomarem Quecksilber zerlegt wird. Der zweite Heizbereich weist hierfür vorteilhaft einen Katalysator auf. Der Katalysator ist dabei dazu ausgebildet, Quecksilber als Dampf passieren zu lassen und Störparameter (Interferenzstoffe) herauszufiltern.

Anschließend können Verbrennungsprodukte in einer Falle in Form eines Amalgams bzw. eines "Amalgam-Kollektors" quantitativ festgehalten und im Weiteren durch Ausheizen spektroskopisch bestimmt werden.

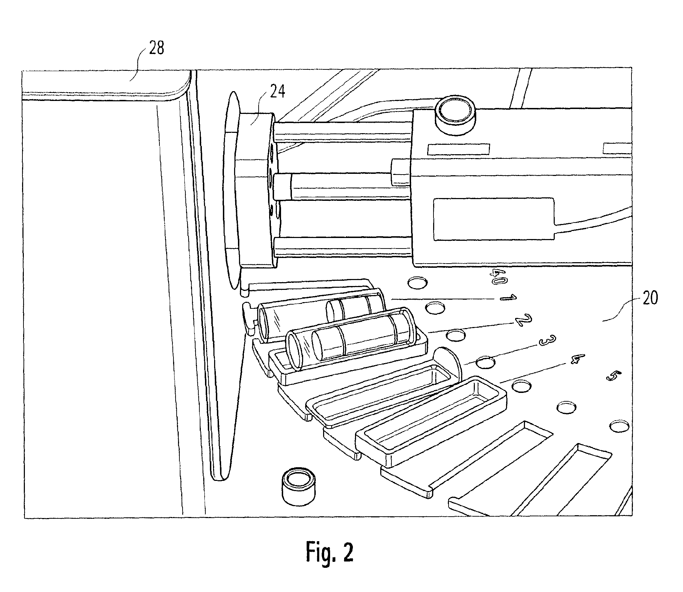

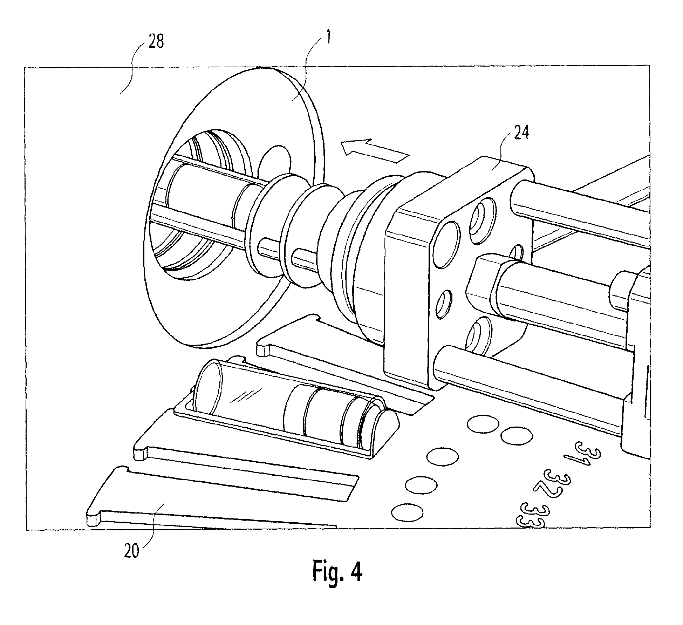

In Fig. 3 ist weiterhin ein Edelstahlkonus 30 zu erkennen, mit dem der Verbrennungsraum verschlossen werden kann. In Fig. 2 ist der Zustand gezeigt, in dem der Verbrennungsraum durch den Edelstahlkonus 30 verschlossen ist. Fig. 4 zeigt den Zustand, in dem der Behälter 1 gerade in den Verbrennungsraum in dem Ofen 28 mithilfe des Transportarms 24 eingebracht wird.

Alternativ zu einem Durchsetzen der Probe durch den Behälter 1 kann auch ein festes oder zähflüssiges Probenmaterial direkt in den Behälter 1 zwischen die beiden Filterelemente 10, 12 eingesetzt werden, zum Beispiel eine Lebensmittelprobe oder Erde. Hierbei können sehr große Probeneinwaagen zwischen die Filterelemente 10, 12 in das Gehäuse 2 gefüllt werden, so dass wiederum eine entsprechend große Genauigkeit bei der Analyse erzielt werden kann.

## Patentansprüche

1. Behälter mit einem Trägermaterial-Element (8), das dazu ausgelegt ist, einen Inhaltsstoff einer gasförmigen oder flüssigen Probe aufzunehmen, aufweisend
- ein Gehäuse (2) mit einer ersten Öffnung (4), die dafür vorgesehen ist, die Probe in das Gehäuse (2) einzuführen und mit einer zweiten Öffnung (6), die dafür vorgesehen ist, die Probe zumindest teilweise in rückhaltbarer Konzentration wieder aus dem Gehäuse (2) auszuführen,
wobei das Trägermaterial-Element (8) in dem Gehäuse (2) angeordnet ist,
**gekennzeichnet durch**
- ein erstes Filterelement (10) und
- ein zweites Filterelement (12),
wobei das erste Filterelement (10), das zweite Filterelement (12) und das Trägermaterial-Element (8) derart in dem Gehäuse (2) angeordnet sind, dass die **durch** die erste Öffnung (4) eingeführte Probe ausschließlich nach einem Durchströmen des ersten Filterelements (10), des Trägermaterial-Elements (8) und des zweiten Filterelements (12) **durch** die zweite Öffnung (6) das Gehäuse (2) verlassen kann.

2. Behälter nach Anspruch 1,
bei dem es sich bei dem Inhaltsstoff um Quecksilber handelt, so dass das Trägermaterial-Element (8) dazu ausgelegt ist, Quecksilber aus der Probe aufzunehmen.

3. Behälter nach Anspruch 1 oder 2,
bei dem das Trägermaterial-Element (8) dazu ausgelegt ist, Quecksilber aus der Probe in Form von Quecksilberatomen und/oder in Form einer Quecksilberverbindung aufzunehmen.

4. Behälter nach einem der vorhergehenden Ansprüche, bei dem das Trägermaterial-Element (8) ein Trägermaterial in Form von Graphit und/oder einen Amalgam-Bildner aufweist.

5. Behälter nach einem der vorhergehenden Ansprüche, bei dem das Gehäuse (2) aus einem sauerstoffresistentem Material besteht, vorzugsweise aus Quarz, Keramik oder Metall.

6. Behälter nach einem der vorhergehenden Ansprüche, bei dem das Gehäuse (2) aus einem Material besteht, das bis 900°C, vorzugsweise bis 1000°C temperaturresistent ist.

7. Behälter nach einem der vorhergehenden Ansprüche, bei dem das erste Filterelement (10) und/oder das zweite Filterelement (12) Quarzfasern und/oder Keramikfasern aufweisen oder aus Quarzfaserwolle und/oder Keramikfaserwolle bestehen.

8. Behälter nach einem der vorhergehenden Ansprüche, bei dem das erste Filterelement (10) und/oder das zweite Filterelement (12) aus einem Material bestehen, das bis 900°C, vorzugsweise bis 1000°C temperaturresistent ist.

9. Behälter nach einem der vorhergehenden Ansprüche, bei dem das Trägermaterial-Element (8) zwischen dem ersten Filterelement (10) und dem zweiten Filterelement (12) angeordnet ist.

10. Behälter nach einem der vorhergehenden Ansprüche, aufweisend wenigstens ein weiteres Filterelement.

11. Vorrichtung zur Bestimmung eines Inhaltsstopffes einer Probe, aufweisend
- einen beheizbaren Verbrennungsraum und
- einen Behälter nach einem der vorhergehenden Ansprüche,
wobei der Behälter und der Verbrennungsraum derart dimensioniert sind, dass sich der Behälter vollständig in den Verbrennungsraum einführen lässt.

12. Vorrichtung nach Anspruch 11,
die dazu ausgebildet ist, das in den Verbrennungsraum eingebrachte Trägermaterial-Element (8) unter Sauerstoffzufuhr zu verbrennen und ein dabei entstehendes Pyrolysegas zu analysieren.
